# FASCICULE DE BREVET EUROPEEN

(11) **EP 2 483 222 B1**
(45) Date de publication et mention de la délivrance du brevet: **11.12.2013**
(21) Numéro de dépôt: 10771799.3
(22) Date de dépôt: 28.09.2010
(51) Int. Cl.: C06B 21/00, C06B 25/34, C07D 487/22

(54) **SUSPENSIONS DE CRISTAUX D'HEXANITROHEXAAZAISOWURTZITANE, OBTENTION DESDITES SUSPENSIONS ET FABRICATION D'OBJETS PYROTECHNIQUES.**
SUSPENSIONEN VON HEXANITROHEXAAZAISOWURTZITANKRISTALLEN, HERSTELLUNG BESAGTER SUSPENSIONEN UND HERSTELLUNG PYROTECHNISCHER OBJEKTE
SUSPENSIONS OF HEXANITROHEXAAZAISOWURTZITANE CRYSTALS, PRODUCTION OF SAID SUSPENSIONS AND PRODUCTION OF PYROTECHNIC OBJECTS

(30) Priorité: 29.09.2009 FR 0956736
(43) Date de publication de la demande: 08.08.2012
(73) Titulaire: Eurenco, 75009 Paris (FR); HERAKLES, 33185 Le Haillan (FR)
(72) Inventeur: MUSCATELLI, Florent, 91710 Vert Le Petit (FR); LESCOP, Philippe, 91710 Vert Le Petit (FR)
(74) Mandataire: Le Roux, Martine
(86) Numéro de dépôt international: PCT/FR2010/052027
(87) Numéro de publication internationale: WO 2011/039459

(56) Documents cités:
- EP-A1- 0 913 374
- EP-A1- 1 327 633
- FR-A1- 2 858 620
- HOLTZ VON E ET AL: "THE SOLUBILITY OF EPSILON-CL-20 IN SELECTED MATERIALS", PROPELLANTS, EXPLOSIVES, PYROTECHNICS, WILEY - V C H VERLAG GMBH & CO., WEINHEIM, DE LNKD- DOI:10.1002/PREP.19940190410, vol. 19, no. 4, 1 janvier 1994 (1994-01-01), pages 206-212, XP009052404, ISSN: 0721-3115
- M.G. Costello et al.: "Fluoroethers and Fluoroamines", , vol. 11 15 octobre 2004 (2004-10-15), pages 877-888, XP002591776, Extrait de l'Internet: URL:http://mrw.interscience.wiley.com/emrw /9780471238966/kirk/article/fluoflyn.a01/c urrent/pdf [extrait le 2010-07-13]
- TOCHIGI K ET AL: "Determination of ASOG parameters for selecting azeotropic mixtures containing hydrofluoroethers", FLUID PHASE EQUILIBRIA 20020330 ELSEVIER NL, vol. 194-197, 30 mars 2002 (2002-03-30), pages 653-662, XP002591777, DOI: DOI:10.1016/S0378-3812(01)00673-2

## Description

La présente invention a pour objet :
- des suspensions de cristaux d'hexanitrohexaazaisowurtzitane dans une phase liquide (renfermant un non solvant original dudit hexanitrohexaazaisowurtzitane),
- un procédé pour l'obtention desdites suspensions, et
- la fabrication d'objets pyrotechniques utilisant de telles suspensions.

L'invention se situe dans le domaine des poudres, propergols et explosifs, utilisés notamment dans les industries d'armement.

Il existe, depuis quelques années, de nombreuses publications relatives au 2,4,6,8,10,12-hexanitro-2,4,6,8,10,12-hexaazatétracyclo (5.5.0.0^{5,9}.0^{3,11}) dodécane, encore appelé hexanitrohexaazaisowurtzitane ou CL20. Ces publications décrivent les diverses formes polymorphes de ce composé (il est en effet connu que l'hexanitrohexaazaisowurtzitane peut être obtenu sous quatre formes polymorphes cristallines : beta, alpha, gamma et epsilon), les propriétés physiques, chimiques et détoniques de ce composé ainsi que l'utilisation dudit composé dans des compositions explosives, des propergols ou des poudres pour armes.

C'est la forme polymorphe epsilon dudit composé (CL20 ε) qui possède la masse volumique la plus élevée (2,04 g/cm³) et qui présente donc le plus d'intérêt, notamment pour utilisation dans les compositions pyrotechniques.

La demande de brevet EP 0 913 374 décrit un procédé d'obtention du CL20 ε selon les étapes réactionnelles suivantes :
- on réalise tout d'abord une solution saturée de CL20 de forme polymorphe quelconque, de préférence autre que la forme epsilon, dans un mélange comprenant, d'une part, un solvant organique du CL20 choisi dans le groupe constitué par les esters, les nitriles, les éthers, les cétones autres que l'acétone et leurs mélanges, et, d'autre part, un non solvant du CL20 choisi dans le groupe constitué par les hydrocarbures aliphatiques, les hydrocarbures aromatiques et leurs mélanges, ledit solvant du CL20 étant plus volatil que ledit non solvant (i.e. présentant une pression de vapeur saturante inférieure à celle du non solvant), et ledit solvant et ledit non solvant étant miscibles dans les proportions utilisées (pour la réalisation dudit mélange, de ladite solution),
- on ensemence ensuite cette solution saturée par quelques cristaux de CL20 ε, puis
- on concentre la solution par évaporation, totale ou partielle, du solvant, ce qui provoque l'apparition de cristaux de CL20 ε qui demeurent en suspension dans le mélange enrichi en non solvant.

Ces cristaux peuvent ensuite être récupérés, par toute méthode usuelle telle que la filtration.

Comme exemples de solvants organiques du CL20, utilisables selon ledit procédé, on peut citer le formiate de méthyle, l'acétate de méthyle, l'acétate d'éthyle, l'acétate d'isopropyle, l'acétonitrile, les mélanges acétate d'éthyle-acétonitrile, le tétrahydrofurane (THF) et la méthyléthylcétone.

Comme exemples de non solvants du CL20, on peut citer le toluène, les xylènes, les alcanes tels que l'hexane, l'heptane et l'octane ainsi que les hydrocarbures aliphatiques halogénés, notamment les hydrocarbures aliphatiques chlorés tels que le 1,2-dichloroéthane.

Le couple solvant/non solvant : acétate d'éthyle/toluène est particulièrement préféré.

Le rapport volumique solvant/non solvant est généralement compris entre 10/90, avantageusement entre 15/85 et 35/65.

Lors de la concentration de la solution par évaporation du solvant organique, la température n'excède avantageusement pas 50°C, ce, en référence à la pureté des cristaux de CL20 ε recherchés.

A l'issue de la mise en oeuvre du procédé selon EP 0 913 374, on obtient donc une suspension de cristaux de CL20 ε dans un non solvant ou un mélange solvant/non solvant enrichi en non solvant, ledit non solvant étant choisi parmi les hydrocarbures aliphatiques, les hydrocarbures aromatiques et leurs mélanges, consistant notamment en au moins un des composés listés ci-dessus, plus particulièrement en le toluène. De tels non solvants sont inflammables.

Ainsi, bien que donnant satisfaction sur le plan de la production industrielle de CL20, le procédé selon EP 0 913 374 présente l'inconvénient d'utiliser un non solvant inflammable. Ceci complique la mise en oeuvre dudit procédé et, plus particulièrement, celle des phases ultérieures audit procédé : phases de conservation, transport et utilisation du CL20 produit (lesdites phases de conservation et de transport n'étant, dans l'absolu, qu'éventuelles). En fin de procédé, le CL20 ne peut donc être conservé en suspension dans les non solvants (ou dans les mélanges solvant/non solvant) retenus dans la demande EP 0 913 374. De ce fait, en fin de procédé selon EP 0 913 374, la suspension de CL20 ε est généralement lavée à l'eau, de façon à éliminer ledit non solvant (ou ledit mélange). Le CL20 produit est ensuite généralement flegmatisé avec de l'eau (à un taux d'environ 20 %) pour son transport et sa conservation. L'élimination ultérieure de l'eau, lors de la fabrication d'objets pyrotechniques, est complexe (elle est généralement mise en oeuvre par séchage en lit) et les traces d'eau résiduelles sont susceptibles d'induire des réactions indésirables avec certains ingrédients de la formulation desdits objets pyrotechniques, notamment avec les agents de réticulation du liant tels que les polyisocyanates.

Dans un tel contexte, il est apparu opportun aux inventeurs de rechercher un non solvant du CL20, ininflammable, adapté à la mise en oeuvre du procédé selon la demande EP 0 913 374 pour l'obtention de suspensions de cristaux de CL20 (de forme polymorphe ε ou autre) et avantageusement optimisé en référence à la mise en oeuvre de la phase ultérieure ou des phases ultérieures audit procédé : la phase d'utilisation desdites suspensions pour la fabrication d'objets pyrotechniques et, généralement, la(les) phase(s) préalable(s) à ladite utilisation, à ladite fabrication (phase(s) de conservation ou(et) de transport), évoquée(s) ci-dessus. Le cahier des charges d'un tel composé, optimisé en référence à toutes les phases indiquées, contient les spécifications ci-après :
- être, bien évidemment, un non solvant du CL20, qui convient à la réalisation d'une solution selon l'enseignement de la demande EP 0 913 374,
- être ininflammable,
- avoir une action flegmatisante sur la charge de CL20 en suspension et ainsi permettre la conservation et le transport sans risque de ladite charge en suspension de CL20, et
- pouvoir être utilisé dans un procédé de fabrication d'objets pyrotechniques (c'est-à-dire d'extraction aisée, au cours de la fabrication de tels objets pyrotechniques et inerte, s'il subsiste à l'état de traces dans l'objet pyrotechnique fabriqué), et idéalement,
- être peu toxique pour l'environnement (contrairement au toluène, utilisé à ce jour).

En référence à un tel cahier des charges, la présente invention propose un perfectionnement à l'enseignement de la demande de brevet EP 0 913 374.

Selon son premier objet, la présente invention concerne des suspensions originales de cristaux d'hexanitrohexaazaisowurtzitane dans une phase liquide. Ces suspensions sont du type de celles obtenues à l'issue de la mise en oeuvre du procédé selon EP 0 913 374. De façon caractéristique, leur phase liquide est constituée à au moins 85 % en masse d'un non solvant de l'hexanitrohexaazaisowurtzitane, ledit non solvant consistant en au moins un hydrofluoroéther ininflammable, et renferme de 0 à moins de 15 % en masse d'un solvant organique de l'hexanitrohexaazaisowurtzitane, plus volatil que ledit non solvant (i.e présentant une pression de vapeur saturante inférieure à celle dudit non solvant), choisi dans le groupe constitué par les esters, les nitriles, les cétones et leurs mélanges. Lesdits solvant (lorsqu'un tel solvant est présent (en tout état de cause, à moins de 15 % en masse)) et non solvant sont miscibles dans les proportions dans lesquelles ils interviennent au sein des suspensions de l'invention. Ledit au moins un hydrofluoroéther ininflammable se substitue ainsi aux hydrocarbures aliphatiques, hydrocarbures aromatiques et leurs mélanges, utilisés comme non solvant dans le procédé selon EP 0 913 374.

Les suspensions de l'invention sont donc susceptibles d'exister selon deux variantes : leur phase liquide comprend ou ne comprend pas (quasiment pas) de solvant de l'hexanitrohexaazaisowurtzitane. Selon une première variante, elles renferment une quantité significative (supérieure ou égale à 4 % en masse) de solvant, toutefois inférieure à 15 % en masse. Ledit solvant est avantageusement choisi parmi l'acétate d'éthyle et l'acétone et consiste très avantageusement en l'acétate d'éthyle. Selon une autre variante, les suspensions de l'invention ne renferment pas ou très peu (moins de 4% en masse, on peut parler de traces) de solvant. Ledit au moins un hydrofluoroéther ininflammable constitue leur phase liquide.

Les hydrofluoroéthers (HFE) sont des composés chimiques constitués d'atomes d'hydrogène, de fluor et de carbone (ils ne contiennent ni chlore, ni brome, ni iode), avec une structure d'éther. Ils sont connus de l'homme du métier. Dans le contexte de la présente invention, ont été retenus les hydrofluoroéthers (HFE) ininflammables. De tels hydrofluoroéthers (HFE) ininflammables sont avantageusement choisis parmi ceux dont l'usage est préconisé dans les compositions de nettoyage à sec selon la demande WO 00/36206, i.e. ceux décrits dans la demande WO 96/22356 et ceux décrits dans le brevet US 6,658,962. Les HFEs sont, de manière générale, connus pour être ininflammables si leur nombre d'atomes de fluor divisé par la somme du nombre d'atomes hydrogène et du nombre de liaisons carbone-carbone est supérieur ou égal à 0,8. A ce propos, on peut se référer à l'enseignement de ladite demande WO 00/36206. Les suspensions de la présente invention renferment donc, à titre de non solvant, un HFE ou un mélange de HFEs présentant cette caractéristique.

Les HFEs ininflammables préférés de l'invention ont une température d'ébullition comprise entre 40°C et 275°C, avantageusement supérieure à 100°C, afin de permettre la conservation, le transport et l'utilisation des suspensions de l'invention dans des conditions de température similaires à celles de suspensions aqueuses. Lesdits HFEs ininflammables, comme l'ensemble des HFEs :
- présentent une faible tension de vapeur saturante et sont ainsi bien adaptés pour l'obtention des suspensions de l'invention par un procédé de cristallisation solvant-non solvant incluant l'évaporation dudit solvant plus volatil que ledit non solvant (voir ci-après le deuxième objet de l'invention) ;
- sont des solvants inertes et à faible chaleur de latente de vaporisation (très inférieure à celle de l'eau) ; ce qui permet l'utilisation directe des suspensions de l'invention, comme matières premières pour la fabrication de composés solides pyrotechniques (voir ci après le troisième objet de l'invention).

Parmi les HFEs ininflammables, ceux de type ségrégué (les "segregated hydofluorether" ininflammables) sont préférés. Ces composés ont été décrits dans les demandes WO 96/22 356 et WO 00/36 206. Leur structure comprend au moins un composé perfluoroalcane, perfluorocycloalcane, perfluoroalcane renfermant un groupe perfluorocycloalkyle, ou perfluoroalcane renfermant un groupe perfluorocycloalkylène, ledit composé étant mono-, di- ou tri- substitué par un groupe alcoxy.

Parmi les HFEs ininflammables, les HFEs ségrégués ininflammables, le 2-trifluorométhyl-3-éthoxy-1,1,1,2,3,4,4,5,5,6,6,6-dodécafluorohexane (dénommé plus simplement ci-après le 2-trifluorométhyl-3-éthoxydodécafluorohexane), de formule brute (CF₃)C₆F₁₂OC₂H₅ est plus particulièrement préféré. Il est référencé sous le numéro CAS 297730-93-9. Sa température d'ébullition est de 128 °C et sa pression de vapeur saturante de 6 mmHg à 20°C. Ses propriétés thermodynamiques lui permettent d'être utilisé dans le cadre de la mise en oeuvre du procédé selon EP 0 913 374, en lieu et place du solvant organique (inflammable) indiqué. Ce composé chimique est notamment produit par la société 3M et commercialisé par cette société sous la dénomination commerciale HFE 7500. La solubilité de l'hexanitrohexaazaisowurtzitane dans le 2-trifluorométhyl-3-éthoxydodécafluorohexane est de 0,012 % en masse à 20 °C, c'est donc bien un non solvant du CL20.

Les HFEs ininflammables (non solvants) sont suffisamment miscibles avec les esters, les nitriles, les cétones et leurs mélanges (solvants), pour l'obtention d'un mélange (au sens de la demande EP 0 913 374), pour l'obtention du résultat recherché.

Les inventeurs ont par ailleurs mis en évidence (voir le point C des exemples ci-après) que les HFEs ininflammables ont un effet flegmatisant avéré de la charge de cristaux CL20 pour un taux massique d'au moins 35%, avantageusement d'au moins 50%. Les suspensions de l'invention renferment donc avantageusement au moins 35% en masse de ce non solvant original (pour au plus 65 % en masse de charge (de cristaux) de CL20), très avantageusement au moins 50 % en masse de ce non solvant original (pour au plus 50 % en masse de charge (de cristaux) de CL20).

Lesdits HFEs ininflammables développent donc une action flegmatisante. Lesdits HFEs ininflammables se substituent ainsi avantageusement aux non solvants du procédé selon EP 0 913 374 et à l'eau, non solvant habituellement utilisé comme flegmatisant pour la conservation et le transport du CL20 (voir ci-dessus). Les suspensions de l'invention peuvent donc, si nécessaire, être conservées et transportées sans risque.

L'homme du métier a déjà, sans nul doute, saisi le grand intérêt des suspensions de l'invention.

Notons incidemment que les suspensions de l'invention renferment des cristaux de CL20 d'une forme polymorphe quelconque, qu'elles renferment avantageusement des cristaux de CL20 ε.

Selon son deuxième objet, la présente invention concerne un procédé d'obtention de telles suspensions. Il s'agit d'un procédé par analogie du procédé selon EP 0 913 374. Il constitue un perfectionnement audit procédé. De façon caractéristique, il est mis en oeuvre avec un non solvant original, ledit au moins un HFE ininflammable. Le procédé, d'obtention d'une suspension de cristaux de CL20 dans un non solvant, de l'invention comprend :
- la préparation d'une solution saturée de CL20 de forme polymorphe quelconque dans un mélange comprenant, d'une part, un solvant organique du CL20 choisi dans le groupe constitué par les esters, les nitriles, les cétones et leurs mélanges, et, d'autre part, un non solvant du CL20 consistant en au moins un HFE ininflammable, ledit solvant du CL20 étant plus volatil que ledit non solvant, (lesdits solvant et non solvant étant évidemment, dans les proportions où ils interviennent, miscibles, pour la constitution desdits mélanges, pour la constitution de ladite solution saturée),
- l'ensemencement de cette solution saturée par quelques cristaux d'hexanitrohexaazaisowurtzitane, puis
- la concentration de la solution ensemencée, par évaporation, totale ou partielle, du solvant.

Avantageusement, l'ensemencement de ladite solution saturée est réalisée par des cristaux d'hexanitrohexaazaisowurtzitane de forme polymorphe ε, afin d'obtenir en fin de procédé une suspension de cristaux d'hexanitrohexaazaisowurtzitane de forme polymorphe ε dans une phase liquide. Selon cette variante de mise en oeuvre, ladite solution est de préférence saturée de CL20 d'une forme polymorphe autre que la forme epsilon.

Ledit procédé est donc caractérisé en ce que ledit non solvant (associé à un solvant avec lequel il est miscible) consiste en au moins un HFE ininflammable, avantageusement en au moins un HFE ininflammable ségrégué, très avantageusement en le 2-trifluorométhyl-3-éthoxydodécafluorohexane

Les teneurs en solvant (moins de 15 % en masse) et cristaux (en suspension) de la suspension obtenue dépendent bien évidemment du mode exact de mise en oeuvre de l'étape de concentration (du taux d'évaporation du solvant recherché). Si l'on vise à obtenir des suspensions quasi exemptes voire exemptes de solvant, le procédé de l'invention comprend, avantageusement, en outre (à l'issue de la mise en oeuvre de l'étape de concentration), le lavage, avec ledit au moins un HFE ininflammable, de la suspension obtenue à l'issue de la concentration de la solution ensemencée.

Avantageusement, le solvant utilisé dans le procédé de l'invention est choisi parmi l'acétate d'éthyle et l'acétone. Très avantageusement, ledit solvant est l'acétate d'éthyle.

Les suspensions de l'invention conviennent par ailleurs parfaitement comme ingrédients (sources de la charge de CL20) dans les procédés de fabrication d'objets pyrotechniques. Elles se prêtent en effet à une utilisation directe dans de tels procédés de fabrication d'objets pyrotechniques. Contrairement à l'eau, utilisée habituellement comme non solvant flegmatisant, lesdits HFEs ininflammables peuvent être aisément extraits par évaporation lors de la mise en oeuvre desdits procédés de fabrication d'objets pyrotechniques. Leur chaleur latente de vaporisation (88 kJ/kg pour le 2-trifluorométhyl-3-éthoxydodécafluorohexane) est beaucoup plus faible que celle de l'eau (2250 kJ/kg) et rend possible leur extraction par distillation au cours des procédés de fabrication des objets pyrotechniques (au contraire, l'extraction de l'eau doit impérativement être réalisée par séchage avant la mise oeuvre du procédé de fabrication). Après extraction, ledit au moins un HFE ininflammable, qui reste en quantités résiduelles, est inerte et n'interagit pas avec les ingrédients des objets pyrotechniques (contrairement à l'eau susceptible de réagir même en très faible quantité avec lesdits ingrédients).

Selon son troisième objet, la présente invention concerne donc la fabrication d'objets pyrotechniques renfermant des cristaux d'hexanitrohexaazaisowurtzitane. Le procédé de fabrication, selon l'invention, desdits objets comprend, de façon classique, l'introduction et le malaxage des ingrédients (liant, agent de réticulation, charge(s)...) dans un malaxeur (pour la constitution d'une pâte). De façon caractéristique, dans le cadre dudit procédé, les cristaux de CL20 ne sont pas introduits, après avoir été séchés, mais au sein d'une suspension selon le premier objet de la présente invention. Ladite suspension est utilisée comme source desdits cristaux, comme ingrédient ou matière première... Ainsi, de façon caractéristique, le procédé de fabrication d'objets pyrotechniques de l'invention comprend :
- l'introduction d'une suspension de l'invention dans le malaxeur, puis, en aval,
- l'extraction sous vide, totale ou quasi-totale, de la phase liquide de ladite suspension.

Les cristaux de CL20 sont introduits dans le malaxeur, au sein d'une suspension (de l'invention), i.e. mouillés (par ledit au moins HFE ininflammable), flegmatisés (par ledit au moins HFE ininflammable), et la phase liquide de ladite suspension (constituée majoritairement, voire essentiellement, voire même exclusivement dudit au moins un HFE ininflammable) est ensuite évacuée par extraction, extraction sous vide, mise en oeuvre sur la pâte malaxée.

Avantageusement, pour faciliter la mise en oeuvre de l'étape d'extraction sous vide de ladite phase liquide, on préfère, parmi les HFEs ininflammables, ceux présentant la plus faible pression de vapeur saturante.

Le grand avantage des suspensions de l'invention est donc qu'elles peuvent être conservées, transportées et utilisées pour la fabrication d'objets pyrotechniques, telles qu'elles (avec le non solvant (voire avec ledit non solvant et de faibles quantités de solvant) utilisé(s) au cours de leur obtention (par un processus solvant/non solvant)).

On a compris que les suspensions de l'invention sont opportunément utilisées pour la fabrication d'objets pyrotechniques et que généralement, avant une telle utilisation (avant leur introduction dans le malaxeur), elles sont conservées ou transportées ou, successivement, conservées puis transportées ou, successivement, transportées puis conservées.

L'invention est maintenant illustrée, de façon nullement limitative, par les figures annexées et exemples ci-après (étude de solubilité, mise en oeuvre du procédé de l'invention, caractérisation d'une suspension de l'invention montrant l'effet flegmatisant du 2-trifluoro-méthyl-3-éthoxydodécafluorohexane et mise en oeuvre d'une extraction du non solvant original de l'invention en mélange avec un liant (extraction telle que celle pouvant être réalisée dans un procédé de fabrication d'objets pyrotechniques).

Le 2-trifluorométhyl-3-éthoxydodécafluorohexane utilisé est le HFE 7500 commercialisé par la société 3 M.
La figure 1 montre la solubilité du CL20 dans un mélange acétate d'éthyle/HFE 7500.
La figure 2A montre une image de microscopie électronique à balayage, la figure 2B une image (à plus fort grossissement) de microscope optique, de cristaux de CL20 ε obtenus par le procédé de l'invention (voir le point B ci-après)
La figure 3 montre les courbes d'évaporation, en évaporateur rotatif, du HFE 7500 en mélange avec un liant (voir le point D ci-après).

### Point A : Etude préalable de la solubilité de l'hexanitrohexaazaisowurtzitane dans le HFE 7500 et dans un mélange acétate d'éthyle/HFE 7500

L'acétate d'éthyle et le HFE 7500 sont totalement miscibles.

On étudie la solubilité du CL20 dans le non solvant HFE 7500 et dans une solution constituée d'un mélange d'acétate d'éthyle (solvant du CL20) et de HFE 7500 (non solvant du CL20).
- La solubilité du CL20 dans le non solvant HFE 7500 a été mesurée, à 20°C, par chromatographie en phase liquide (HPLC), à 0,012 g/100 g de HFE 7500.
- En référence aux procédés de cristallisation du CL20 de l'art antérieur (procédés développés à l'échelle industrielle), il est intéressant de cibler une solubilité d'environ 100 g de CL20 dissout par litre de la solution constituée d'un mélange d'acétate d'éthyle (solvant du CL20) et de HFE 7500 (non solvant du CL20).

Des essais conduits à petite échelle (volume de solution inférieur à 100 mL), à 20°C, présentés dans le tableau 1, ont tout d'abord permis de définir sommairement un intervalle de rapport de mélange adéquat.

**Tableau 1**

| % vol en acétate d'éthyle | % vol en HFE 7500 | Solubilité du CL20 en g/L (20°C) |
|---|---|---|
| 40 | 60 | 44 |
| 45 | 55 | 75 |
| 50 | 50 | 155 |

Une solubilité d'environ 100 g par litre de CL20 est obtenue pour un mélange comprenant 45 à 50% en volume d'acétate d'éthyle.

Le choix s'est donc orienté vers un mélange acétate d'éthyle/HFE 7500 à 47/53 % en vol pour une étude de solubilité plus approfondie en réacteur thermostaté d'une capacité de 5L. Le suivi de la solubilisation, pas à pas, a été réalisé au moyen d'une sonde LASENTEC^{®} *in situ.*

On prépare donc 1,5 L de solution acétate d'éthyle/HFE 7500 à 47/53 % vol (soit respectivement 634,5 g d'acétate et 1293,4 g de HFE 7500) que l'on thermostate à 20°C. On additionne alors par petites quantités le CL20 et on suit sa dissolution par l'intermédiaire des mesures de la sonde *in situ.* La quantité de CL20 que l'on peut dissoudre dans le mélange est de 143 g, ce qui représente donc une solubilité de 95,3 g par litre de solution non saturée.

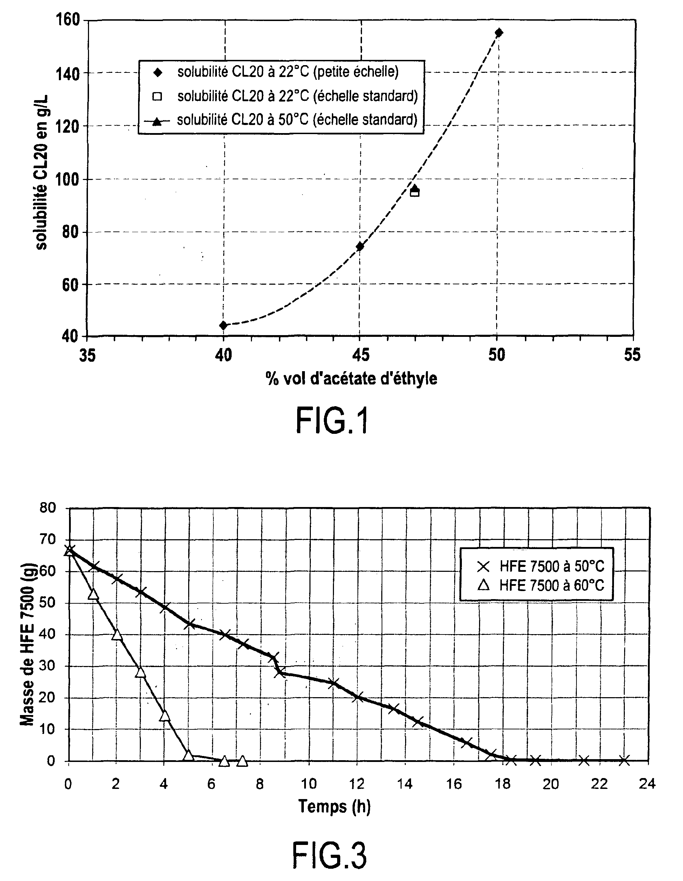

On chauffe le mélange à 50°C et on répète le mode opératoire précédent pour l'évaluation de la solubilité. La solubilité est mesurée à 96,6 g/L de solution. Elle est donc quasiment invariante avec la température pour le mélange donné acétate d'éthyle/HFE 7500 47/53 % vol. Les différentes mesures effectuées sont reportées sur le graphe de la figure 1.

### Point B : Mise en oeuvre du procédé de l'invention (exemple)

Suivant l'enseignement du point A ci-dessus, on réalise, dans un réacteur d'un volume de 2 L, une cristallisation par évaporation à partir d'un milieu acétate d'éthyle/HFE 7500 à 47/53 % vol comprenant 470 mL d'acétate d'éthyle et 530 mL de HFE 7500 saturé en CL20 (soit avec 95,3 g de CL20 dissous).

On trouve comme élément particulier (sur un montage de distillation classique), le mobile d'agitation (hélice Lightnin A 310) qui possède un profil mince, peu brisant, avec cependant une bonne capacité de pompage, permettant de pallier à la décantation des cristaux dans des régimes de rotation tout en évitant la brisure en bout de pale. Le diamètre de l'agitateur par rapport au diamètre de cuve est de 64 mm/100 mm. Son positionnement dans le réacteur (distance entre l'agitateur et le fond de cuve) a été également défini par les règles du génie chimique. Le taux de remplissage du réacteur ne dépasse pas la moitié. Tout ceci permet, lors de l'opération, de continuellement faire passer les cristaux en phase de croissance à l'interface d'évaporation (lieu où la solution se désature) sans trop les solliciter "mécaniquement".

Après introduction, dans le réacteur, de la solution de départ saturée en CL20, on porte la température de masse à 60°C puis on introduit la semence (10 g de CL20 ε de taille moyenne 30 µm). La vitesse d'agitation est de 480 tr/min (ce qui correspond à une puissance par unité de volume, P/V = 0,21 W/L, à une vitesse en périphérie du mobile d'agitation, Vp = 1,6 m/s, et à un débit de pompage, Qp = 1,2 L/s). On distille alors progressivement pendant 1h45 l'acétate d'éthyle par ajustement d'un taux de reflux approprié et d'une rampe de vide adaptée de 700 à 490 mbar, fonction de la composition au bouilleur. A l'issue de l'épuisement complet en acétate d'éthyle au bouilleur, on revient à pression atmosphérique et on vidange la suspension de CL20 ε dans HFE 7500.

Pour analyser, par microscopie électronique, les cristaux de CL20 ε de la suspension, la suspension est filtrée et le rétentat essoré. Le produit est ensuite séché en étuve.

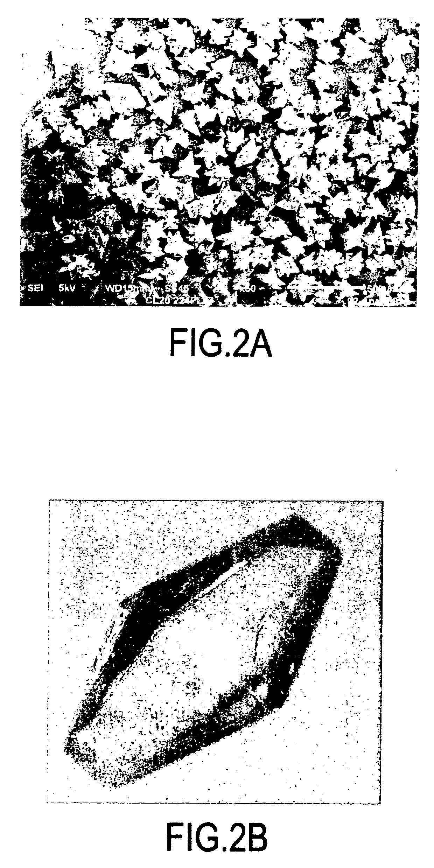

On récupère sur le filtre un produit cristallin, de couleur "blanc cassé", composé de cristaux de CL20 ε d'une taille moyenne de 100 µm, tels que montrés sur les figures 2A et 2B.

### Point C : Flegmatisation du CL20 ε en suspension dans le HFE 7500

Le tableau 2 ci-après présente les caractérisations de la flegmatisation du CL20 ε par le HFE 7500, flegmatisation étudiée sur des mélanges HFE 7500/CL20 ε, avec du CL20 ε de granulométrie 35 ± 15 µm, à différents taux massiques. La flegmatisation a été évaluée par des tests de sensibilité à l'impact *(ISI), sensibilité aux frottements **(ISF), sensibilité à l'amorçage par étincelle électrique (ES)*** et un test de transition déflagration détonation ***(TDD).

**Tableau 2**

| | CL20 ε sec | CL20 ε / HFE 7500 20% en masse | CL20 ε / HFE 7500 35% en masse | CL20 ε / HFE 7500 50% en masse |
|---|---|---|---|---|
| ISI (J) | 1,9 | 1,4 | 7,5 | (7+/30) à 50,1 |
| ISF (N) | 80 | 89 | 83 | 114 |
| ES (mJ) | de 413 à 726 | > 726 | > 726 | > 726 |
| TDD (mm) | 50 (combustion) | 150 (combustion) | >350 (combustion) | >350 (combustion) |
| | 75 (déflagration) | 175 (déflagration) | | |
| | 100 (détonation) | | | |

* Test de sensibilité à l'impact (ISI) : l'épreuve réalisée correspond à celle décrite dans la norme NF T 70-500, elle-même semblable à l'épreuve ONU 3a)ii) issue des "Recommandations relatives au Transport des marchandises dangereuses - manuel d'épreuves et de critères, Quatrième édition révisée, ST/SG/AC.10/11/Rev.4, ISBN 92-1-239083-8ISSN 1014-7179". Par une série minimale de 30 essais, on détermine l'énergie entraînant 50% (méthode de traitement des résultats de Bruceton) de résultats positifs d'une matière explosible soumise aux chocs d'un mouton. La matière à éprouver est confinée dans un dispositif en acier constitué de 2 galets et d'une bague de guidage. En modifiant la masse et la hauteur de chute du mouton, on peut faire varier l'énergie de 1 à 50 J. Compte tenu de la faible quantité de matière disponible pour certains des produits testés, il n'a été réalisé, pour lesdits produits, qu'un nombre réduit d'épreuves de reproductibilité, par rapport aux recommandations de la norme NF T 70-500.

** Test de Sensibilité au frottement (ISF): l'épreuve réalisée correspond à celle décrite dans la norme NF T 70-503, elle-même semblable à l'épreuve ONU 3b)ii). Par une série minimale de 30 essais, on détermine en utilisant la méthode Bruceton, la force entraînant 50% de résultats positifs d'une matière explosible soumise à un frottement. La matière à éprouver est placée sur une plaquette de porcelaine de rugosité définie, animée d'un seul mouvement de va et vient, de 10 mm d'amplitude à la vitesse de 7 cm/s à vide, par rapport à un crayon de porcelaine reposant sur la matière. La force appliquée sur le crayon de porcelaine qui est appuyé sur la matière peut varier de 7,8 à 353 N. Compte tenu de la faible quantité de matière disponible pour certains des produits testés, il n'a été réalisé, pour lesdits produits, qu'un nombre réduit d'épreuves de reproductibilité, par rapport aux recommandations de la norme NF T 70-500.

*** Test de Sensibilité à l'amorçage par étincelle électrique (ES): l'épreuve réalisée est une épreuve mise au point par la Demanderesse sans équivalent NF ou ONU. La matière à éprouver, disposée dans une coupelle de diamètre 10 mm et de hauteur 1,5 mm, est placée entre 2 électrodes et est soumise à une étincelle électrique d'énergie variable de 5 à 726 mJ. On observe s'il y a événement pyrotechnique ou non et on détermine le seuil d'énergie n'assurant plus l'initiation de la matière. Cette valeur est confirmée par 20 essais successifs. Compte tenu de la faible quantité de matière disponible pour certains des produits testés, il n'a été réalisé, pour lesdits produits, qu'un nombre réduit d'épreuves de reproductibilité.

**** Test de transition déflagration détonation (TDD) : cette épreuve sert à déterminer les possibilités de transition d'un explosif contenu dans un tube acier semi clos (de diamètre intérieur 40 mm, d'épaisseur 4 mm et de longueur 200 et/ou 400 mm. La partie inférieure du tube est fermée par soudure par une plaque de même nature de 150x150x4 mm³) suite à une inflammation locale en surface (le dispositif d'allumage est constitué par un fil résistif nickel/chrome (80/20) de 0,4 mm de diamètre et de 5 à 10mm de longueur).

Le résultat de l'essai est apprécié à partir de l'aspect du tube acier. On peut distinguer trois cas :
- Combustion : Tube intact ou gonflé (essai négatif).
- Déflagration : Tube fragmenté en gros éclats (essai positif).
- Détonation : Tube fragmenté avec présence de petits éclats (essai positif).

On enregistre :
- la plus grande hauteur (exprimée en mm) pour laquelle il a été obtenu deux essais négatifs en sachant qu'au pas supérieur (+ 25 mm) il a été obtenu un résultat positif.
- la plus faible hauteur (exprimée en mm) pour laquelle il a été obtenu un essai positif.

Pour 20% de HFE 7500 en masse incorporé à du CL20, l'effet flegmatisant n'est pas démontré. Une des causes probables provient de la difficulté à obtenir un mélange homogène pour un taux de mouillant aussi faible en partant d'un produit sec. Les valeurs obtenues aux différents tests sont proches de celles obtenues sur du CL20 sec.

En revanche, à partir de 35% en masse de HFE 7500, l'effet flegmatisant est plus prononcé. L'ISI est de 7,5 J (contre 1,9 J sur le même CL20 sec) et il y a combustion pour la valeur haute du test de TDD. Il subsiste néanmoins quelques difficultés à préparer un mélange homogène.

A partir de 50%, l'effet flegmatisant est avéré. Les valeurs hautes des différents tests effectués sont atteintes et on note également un effet flegmatisant sur la sensibilité aux agressions de type frottements (114 N contre 80-90 N pour le CL20 sec).

### Point D : Extraction du HFE 7500 en mélange avec un liant, susceptible d'être mise en oeuvre dans un procédé de fabrication d'objets pyrotechniques

La possibilité d'extraire le HFE 7500 en mélange avec un liant de type polyadipate de diéthylène glycol (PADEG) a été évaluée dans un évaporateur rotatif de laboratoire. On simule ainsi l'extraction du HFE 7500 dans un procédé de fabrication d'objets pyrotechniques.

Les tests d'extraction de l'HFE *7500* ont été réalisés sur un mélange contenant en masse 1/3 de PADEG et 2/3 de HFE 7500.

Une masse de 100 grammes de mélange a été placée dans l'évaporateur rotatif. Les conditions opératoires sont reportées ci-dessous :
- pression : 100 - 130 mbars
- température : 50 et 60°C
- temps d'extraction : jusqu'à disparition totale du HFE 7500 par mesure de perte de masse
- relevé de la masse évaporée toute les heures.

Afin de suivre la quantité de HFE 7500 extraite au cours du temps, les ballons sont pesés toutes les heures. L'évolution de la perte de masse du mélange a donc été suivie jusqu'à la disparition quasi-totale de la quantité supposée de HFE 7500.

Le graphe de la figure 3 montre la perte de masse (des mélanges PADEG/HFE 7500) liée à l'évaporation du HFE 7500 pour les deux températures de test (pour un vide équivalent) : la vitesse d'extraction est 3,5 fois plus rapide à 60°C qu'à 50°C, le taux résiduel de HFE 7500 après extraction est inférieur à 0,2% de celui du HFE 7500 initial.

Afin de vérifier avec plus d'exactitude le taux de HFE 7500 présent en fin d'extraction, le mélange récupéré en fin de test réalisé à 50°C a été analysé par chromatographie en phase gazeuse. Le taux résiduel de HFE 7500 mesuré par cette méthode est de 0,02 %.

La possibilité d'extraire le HFE 7500 d'une pâte de propergol en cours de malaxage, au moyen d'un dispositif de malaxage sous vide muni d'un condenseur, a aussi été démontrée.

## Revendications

1. Suspension de cristaux d'hexanitrohexaazaisowurtzitane dans une phase liquide, **caractérisée en ce que** ladite phase liquide est constituée à au moins 85 % en masse d'un non solvant de l'hexanitrohexaazaisowurtzitane, ledit non solvant consistant en au moins un hydrofluoroéther ininflammable, et renferme de 0 à moins de 15 % en masse d'un solvant organique de l'hexanitrohexaazaisowurtzitane, plus volatil que ledit non solvant, choisi dans le groupe constitué par les esters, les nitriles, les cétones et leurs mélanges.

2. Suspension selon la revendication 1, **caractérisée en ce que** ledit solvant, présent à moins de 15 % en masse dans ladite phase liquide, est choisi parmi l'acétate d'éthyle et l'acétone et consiste avantageusement en l'acétate d'éthyle.

3. Suspension selon la revendication 1 ou 2, **caractérisée en ce que** ledit hydrofluoroéther ininflammable est choisi parmi les hydrofluoroéthers ininflammables ségrégués.

4. Suspension selon l'une quelconque des revendications 1 à 3, **caractérisée en ce que** ledit hydrofluoroéther ininflammable consiste en le 2-trifluorométhyl-3-éthoxydodécafluorohexane.

5. Suspension selon l'une quelconque des revendications 1 à 4, **caractérisée en ce qu'**elle renferme au moins 35% en masse, avantageusement au moins 50 % en masse, dudit au moins un hydrofluoroéther ininflammable.

6. Procédé d'obtention d'une suspension de cristaux d'hexanitrohexaazaisowurtzitane selon l'une quelconque des revendications 1 à 5, **caractérisé en ce qu'**il comprend :
- la préparation d'une solution saturée d'hexanitrohexaazaisowurtzitane de forme polymorphe quelconque dans un mélange comprenant, d'une part, un solvant organique de l'hexanitrohexaazaisowurtzitane choisi dans le groupe constitué par les esters, les nitriles, les cétones, et leurs mélanges, et, d'autre part, un non solvant dudit hexanitrohexaazaisowurtzitane consistant en au moins un hydrofluoroéther ininflammable, ledit solvant étant plus volatil que ledit non solvant,
- l'ensemencement de ladite solution saturée par quelques cristaux d'hexanitrohexaazaisowurtzitane, puis
- la concentration de ladite solution ensemencée par évaporation, totale ou partielle, du solvant.

7. Procédé selon la revendication 6, **caractérisé en ce qu'**il comprend, en outre, le lavage, avec ledit au moins un hydrofluoroéther ininflammable, de la suspension obtenue à l'issue de ladite concentration de ladite solution ensemencée.

8. Procédé selon la revendication 6 ou 7, **caractérisé en ce que** ledit solvant est choisi parmi l'acétate d'éthyle et l'acétone et consiste avantageusement en l'acétate d'éthyle.

9. Procédé de fabrication d'objets pyrotechniques renfermant des cristaux d'hexanitrohexaazaisowurtzitane, comprenant l'introduction et le malaxage des ingrédients dans un malaxeur, **caractérisé en ce qu'**il comprend :
- l'introduction d'une suspension selon l'une quelconque des revendications 1 à 5 dans un malaxeur, et
- l'extraction sous vide, totale ou quasi-totale, de la phase liquide de ladite suspension.

10. Procédé selon la revendication 9, **caractérisé en ce qu'**il comprend, en outre :
- la conservation ou le transport ou la conservation et le transport ou le transport et la conservation de ladite suspension, avant son introduction dans ledit malaxeur.

## Patentansprüche

1. Suspension von Hexanitrohexaazaisowurtzitan-Kristallen in einer flüssigen Phase, **dadurch gekennzeichnet, daß** die flüssige Phase zu wenigstens 85 Masseprozent von einem Nichtlösungsmittel des Hexanitrohexaazaisowurtzitan gebildet ist, wobei das Nichtlösungsmittel aus wenigstens einem nicht entzündbaren Hydrofluorether besteht, und 0 bis weniger als 15 Masseprozent eines organischen Lösungsmittels des Hexanitrohexaazaisowurtzitan, das flüchtiger als das Nichtlösungsmittel ist, ausgewählt aus der durch die Ester, die Nitrile, die Ketone und deren Mischungen gebildeten Gruppe, enthält.

2. Suspension nach Anspruch 1, **dadurch gekennzeichnet, daß** das zu weniger als 15 Masseprozent in der flüssigen Phase vorliegende Lösungsmittel aus Ethylacetat und Aceton ausgewählt ist und vorteilhafterweise aus Ethylacetat besteht.

3. Suspension nach Anspruch 1 oder 2, **dadurch gekennzeichnet, daß** der nicht entzündbare Hydrofluorether aus den segregierten nicht entzündbaren Hydrofluorethern ausgewählt ist.

4. Suspension nach einem der Ansprüche 1 bis 3, **dadurch gekennzeichnet, daß** der nicht entzündbare Hydrofluorether aus 2-Trifluormethyl-3-ethoxydodecafluorhexan besteht.

5. Suspension nach einem der Ansprüche 1 bis 4, **dadurch gekennzeichnet, daß** sie wenigstens 35 Masseprozent, vorteilhafterweise wenigstens 50 Masseprozent des wenigstens einen nicht entzündbaren Hydrofluorethers enthält.

6. Verfahren zum Erhalten einer Suspension von Hexanitrohexaazaisowurtzitan-Kristallen nach einem der Ansprüche 1 bis 5, **dadurch gekennzeichnet, daß** es umfaßt:
- die Zubereitung einer gesättigten Lösung von Hexanitrohexaazaisowurtzitan von beliebiger polymorpher Form in einer Mischung, die einerseits ein organisches Lösungsmittel des Hexanitrohexaazaisowurtzitan, ausgewählt aus der durch die Ester, die Nitrile, die Ketone und deren Mischungen gebildeten Gruppe, sowie andererseits ein Nichtlösungsmittel des Hexanitrohexaazaisowurtzitan, das aus wenigstens einem nicht entzündbaren Hydrofluorether besteht, wobei das Lösungsmittel flüchtiger als das Nichtlösungsmittel ist, umfaßt,
- Beimpfen der gesättigten Lösung mit einigen Hexanitrohexaazaisowurtzitan-Kristallen, anschließend
- die Konzentrierung der beimpften Lösung durch vollständiges oder teilweises Verdampfen des Lösungsmittels.

7. Verfahren nach Anspruch 6, **dadurch gekennzeichnet, daß** es ferner das Waschen der am Ende der Konzentrierung der beimpften Lösung erhaltenen Suspension mit dem wenigstens einen nicht entzündbaren Hydrofluorether umfaßt.

8. Verfahren nach Anspruch 6 oder 7, **dadurch gekennzeichnet, daß** das Lösungsmittel aus Ethylacetat und Aceton ausgewählt ist und vorteilhafterweise aus Ethylacetat besteht.

9. Verfahren zur Herstellung von pyrotechnischen Gegenständen, die Hexanitrohexaazaisowurtzitan-Kristalle enthalten, umfassend das Einbringen und das Mischen der Bestandteile in einen/einem Mischer, **dadurch gekennzeichnet, daß** es umfaßt:
- Einbringen einer Suspension nach einem der Ansprüche 1 bis 5 in einen Mischer und
- vollständige oder beinahe vollständige Vakuum-Extraktion der flüssigen Phase der Suspension.

10. Verfahren nach Anspruch 9, **dadurch gekennzeichnet, daß** es ferner umfaßt:
- das Konservieren oder den Transport oder das Konservieren und den Transport oder den Transport und das Konservieren der Suspension vor ihrem Einleiten in den Mischer.

## Claims

1. A suspension of hexanitrohexaazaisowurtzitane crystals in a liquid phase, **characterized in that** said liquid phase is composed, to at least 85% by weight, of a nonsolvent for hexanitrohexaazaisowurtzitane, said nonsolvent consisting of at least one nonflammable hydrofluoroether, and includes from 0 to less than 15% by weight of an organic solvent for hexanitrohexaazaisowurtzitane, which is more volatile than said nonsolvent, chosen from the group consisting of esters, nitriles, ketones and their mixtures.

2. The suspension according to claim 1, **characterized in that** said solvent, present at less than 15% by weight in said liquid phase, is chosen from ethyl acetate and acetone and advantageously consists of ethyl acetate.

3. The suspension according to claim 1 or 2, **characterized in that** said nonflammable hydrofluoroether is chosen from segregated nonflammable hydrofluoroethers.

4. The suspension according to any one of claims 1 to 3, **characterized in that** said nonflammable hydrofluoroether consists of 2-trifluoromethyl-3-ethoxydodecafluorohexane.

5. The suspension according to any one of claims 1 to 4, **characterized in that** it includes at least 35% by weight, advantageously at least 50% by weight, of said at least one nonflammable hydrofluoroether.

6. A process for obtaining a suspension of hexanitrohexaazaisowurtzitane crystals according to any one of claims 1 to 5, **characterized in that** it comprises:
- the preparation of a saturated solution of hexanitrohexaazaisowurtzitane of any polymorphic form in a mixture comprising, on the one hand, an organic solvent for hexanitrohexaazaisowurtzitane chosen from the group consisting of esters, nitriles, ketones and their mixtures and, on the other hand, a nonsolvent for said hexanitrohexaazaisowurtzitane consisting of at least one nonflammable hydrofluoroether, said solvent being more volatile than said nonsolvent,
- the seeding of said saturated solution with a few crystals of hexanitrohexaazaisowurtzitane, then
- the concentrating of said seeded solution by complete or partial evaporation of the solvent.

7. The process according to claim 6, **characterized in that** it additionally comprises the washing, with said at least one nonfilammable hydrofluoroether, of the suspension obtained on conclusion of said concentrating of said seeded solution.

8. The process according to claim 6 or 7, **characterized in that** said solvent is chosen from ethyl acetate and acetone and advantageously consists of ethyl acetate.

9. A process for the manufacture of pyrotechnic objects including hexanitrohexaazaisowurtzitane crystals, comprising the introduction and the kneading of the ingredients in a kneader, **characterized in that** it comprises:
- the introduction of a suspension according to any one of claims 1 to 5 into a kneader, and
- the complete or virtually complete extraction under vacuum of the liquid phase of said suspension.

10. The process according to claim 9, **characterized in that** it additionally comprises:
- the storage or transportation or the storage and transportation or the transportation and storage of said suspension, before it is introduced into said kneader.
